# EUROPEAN PATENT APPLICATION

(11) **EP 4 410 270 A1**
(43) Date of publication of application: **07.08.2024**
(21) Application number: 22874004.9
(22) Date of filing: 03.10.2022
(51) Int. Cl.: A61K 8/30, A61K 8/60, A61K 8/34, A61Q 5/02

(54) **ANTISEBOGENIC COMPOSITION, FORMULATION AND USE OF THE COMPOSITION**

(30) Priority: 01.10.2021 BR 102021019756
(71) Applicant: Chemyunion Ltda, 18087-800 Sorocaba (BR)
(72) Inventor: MUSSI, Lilian, 18013-240 Sorocaba (BR); NOGUEIRA, Cecília, 18085-844 Sorocaba (BR); JUNIOR, Flávio Bueno De Camargo, 18013-004 Avenida São Paulo, 1791, Além Ponte (BR); MAGALHÃES, Wagner Vidal, 18090-360 Sorocaba (BR)
(74) Representative: Grünecker Patent- und Rechtsanwälte PartG mbB
(86) International application number: PCT/BR2022/050387
(87) International publication number: WO 2023/049981

(57) **Abstract**

The present invention describes antisebogenic compositions comprising a synergistic combination of glycols, polyols, and fatty acids. In one embodiment, the present invention shows the use of these compositions in controlling sebum production in the skin and in the treatment of acne. The present invention lies in the fields of Pharmacy, Chemistry, and Cosmetology.

## Description

### Field of the Invention

The present invention describes antisebogenic compositions comprising a synergistic combination of glycols, polyols, and fatty acids. In one embodiment, the present invention shows the use of these compositions in controlling sebum production in the skin and in the treatment of acne. The present invention lies in the fields of Pharmacy, Chemistry, and Cosmetology.

### Background of the Invention

Sebum is a secretion produced by the sebaceous glands. Such secretion is formed by triglycerides, cholesterol, cholesterol esters and fatty acids, in addition to portions of the secretory cell. The function of sebum is to maintain skin hydration through the formation of a protective lipid layer which reduces the loss of fluids through the epidermis. Sebum also lubricates the surface of the skin and prevents hair from drying out (BOELSMA et al, 2003). Despite its beneficial aspects, excessive sebum production can trigger numerous conditions related to the skin and scalp, such as: increased pore size, which results in an aged appearance of the skin; sebaceous hyperplasia, a benign disorder of the sebaceous glands, which increase in size; dermatosis; seborrhea, a disease that causes scaly, red patches on the skin, mainly on the scalp, and whose symptoms may include rash, dandruff, erythema. Sebum production is also directly related to acne pathology.

Acne is a skin condition that affects approximately 9.4% of the world's population, listed in eighth place among the most common skin diseases (TAN & BHATE, 2015; HENG & CHEW, 2020). Very common in adolescents, it is estimated that 85% of this population is affected by this condition which, despite reducing with age, still have a significant prevalence in adulthood, affecting approximately up to 40% of women between 20-40 years old (HOLZMANN & SHAKERY, 2014; HENG & CHEW, 2020). Despite being more common in men during adolescence, this trend is reversed in adult acne cases (KNUTSEN-LARSON et al., 2012).

Acne phenotypes appear more frequently on the face, chest and back, regions where the number of sebaceous glands is greater, and their treatment is directly linked to their grade (I, II, III and IV) (SOCIEDADE BRASILEIRA DE CIRURGIA DERMATOLOGICA, 2019).

Acne disorder arises from a multifactorial condition, which may involve gender, age, ethnicity, genetics, habits, etc. Basically, acne is caused by 4 processes: increased and altered sebum production, colonization of the hair follicle by *Cutibacterium acnes* (*C*. *acnes* - formerly known as *Propionibacterium acnes*), localized release of inflammatory mediators and alteration in the keratinization process that leads to the formation of comedones (WILLIAMS et al, 2012). The order in which the events are triggered is still uncertain, but it is a fact that for acne to appear, all 4 processes must occur.

*C. acnes* is an anaerobic but oxygen-tolerant microorganism resident in the skin microflora. Evidence shows that it is present in significant numbers on human skin, regardless of whether it has acne (JAPPE et al., 2002). This microorganism is predominantly found in areas rich in sebaceous glands and also close to eccrine and mucous sweat glands, as it feeds on lipids and fatty acids, compounds produced in these regions (SHU et al., 2013), secreting porphyrins, molecules which can generate reactive oxygen species, feeding the inflammatory process (PLATSIDAKI et al. 2018).

In fact, colonization of the hair follicle by *C. acnes* is considered one of the central factors of acne (DRENO et al., 2018). Its presence at high levels can also trigger an innate immune reaction in both very early (microcomedogenic) and late (inflammatory) acne lesions through the activation of the toll-like receptor 2 (TLR2) (TANGHETTI, 2013), which triggers a cascade of inflammatory activation that causes irritation, redness and, occasionally, hyperpigmentation in acne lesions, in addition to stimulating the proliferation and abnormal differentiation of keratinocytes, generating hyperkeratinization and, consequently, comedones (KNUTSEN-LARSON et al., 2012; PLATSIDAKI et al ., 2018).

Reports show that around 20-40% of acne cases also seem to indicate the presence of *Staphylococcus aureus* (FANELLI et al. 2011, Khorvash et al. 2012). However, its contribution to the development of acne is still quite controversial and, if so, it is probably related to the amplification of inflammatory symptoms (FANELLI et al. 2011).

In the treatment of acne, by topical application, the most commonly used ingredients are retinoids, benzoyl peroxide, salicylic acid, sulfur, sodium sulfacetamide and alpha-hydroxy acids (AHAs) (BOWE and SHALITA, 2008; BRENNER et al., 2012).

Isotretinoin is a highly potent retinoid that inhibits sebum production. This effect is believed to be linked to the induction of apoptosis in several cell types, including sebocytes in the sebaceous gland (NELSON et al, 2006). However, the list of adverse effects is wide and well known and, therefore, its use in combating oily skin can be considered controversial. Other retinoids, such as tretinoin and adapalene, remain as options, but can cause skin irritation characterized by redness and peeling; furthermore, its use requires medical guidance (CHIEN, 2018).

One of the ingredients most recommended by dermatologists in cases of mild or moderate acne is benzoyl peroxide. Typically applied in lotions, creams and cleansing gels, benzoyl peroxide acts to reduce the population of *C. acnes,* through the generation of reactive oxygen species (ROS) in the pilosebaceous follicle. Therefore, it helps in the dissolution of sebum, preventing the growth of microorganisms (SAGRANSKY et al., 2009). However, it is suggested that to eliminate *C. acnes,* the application of benzoyl peroxide should be combined with a topical antibiotic (BOWE and SHALITA, 2008).

Salicylic acid is a phytohormone that acts in the regulation of skin cell growth and differentiation, promoting the desquamation of the stratum corneum due to its chemical affinity with the intercellular cement between corneocytes. In this way, salicylic acid reduces the corneal layer of the skin and, consequently, unclogs the pilosebaceous follicles, increasing cell renewal (FONSECA et al., 2012).

Similar to salicylic acid, alpha-hydroxy acids (AHAs), from fruits, vegetables and milk, are weak organic acids that promote skin peeling through intercellular ionic bonds in the epidermis, thus reducing the cohesion of corneocytes. Due to their keratolytic property, AHAs are commonly indicated for the treatment of moderate to severe acne. Furthermore, the compound is also used to treat hyperpigmentation, caused by the inflammatory process of acne (BABILAS et al, 2012).

Although the mechanism of action of sulfur in combating skin acne has not been completely elucidated, the application of the chemical element in the treatment of acne has been carried out for decades. It is suggested that through interaction with cysteine in the stratum corneum, sulfur acts as an antimicrobial agent with keratolytic properties, that is, it dissolves the corneal layer of the skin with the aim of reducing the action of *C. acnes* (NETO et al., 2016).

Sodium Sulfacetamide is a compound capable of inhibiting the proliferation of *C. acnes,* via competitive antagonism of para-aminobenzoic acid, preventing the synthesis of bacterial DNA and, consequently, bacterial proliferation (SWANSON, 2003).

Most of the antimicrobials available to combat *C. acnes* are prohibited in cosmetic products, including azelaic acid, clindamycin, erythromycin and tetracycline, as these antimicrobials belong to the class of drugs that can only be obtained by medical prescription. Therefore, there are few alternatives left for acne treatment in cosmetics with the use of antimicrobials. WANNMACHER (2006) and AGOSTINHO (2017) point out that numerous antimicrobials available on the market, although demonstrating efficacy, cause adverse reactions such as: itching, burning sensation and redness of the skin.

The association of the use of ingredients such as antimicrobial agents, antibiotics, exfoliants and aesthetic cleaning techniques, such as the physical removal of comedones and the unclogging of follicles can be combined synergistically to combat acne. Furthermore, some cosmetic ingredients intended for the treatment of acne, such as botanical extracts and complex raw materials (blends) that act as sebum-regulators, keratolytics and anti-inflammatories, can also be alternatives for acne treatment. Leyden (2003) points out that the best way to combat acne is through therapeutic combinations, thus enabling the individual to achieve faster results in an effective manner. Furthermore, Gollnick (2015) indicates that due to multiple pathogenic factors of acne, treatment with combination of ingredients is a rational and effective strategy, resulting in rapid and complete removal of lesions compared to monotherapies.

Propanediol is a glycol, it can be produced from renewable resources using microorganisms. In addition to its action as a humectant for the skin, propanediol is known as a "booster" in preservation systems, having synergistic action with traditional preservatives in concentrations of up to 2% (COSMETIC INNOVATION, 2016).

There is currently no report of propanediol acting against C. *acnes* or significant action in controlling sebum or keratolytic activity. It is described in the literature that propylene glycol (1,2-propanediol) and glycerol have keratolytic activity (FLURH et al., 2008 HAMADA & TAMATE, 1981; ROUSSEL et al., 2012; CORAZZA et al, 2021).

Regarding anti-inflammatory activity, no direct evidence was found that propanediol has such activity, but there is evidence that it is used in formulations (combinations of different components) that have anti-inflammatory activity (MEYER et al, 2014), possibly due to its function as a solvent or even an permeation enhancer of actives and that propanediol derivatives are used to obtain delivery systems for anti-inflammatory active ingredients (TAKEDATSU, et al, 2015). There are studies that report that glycerol, the starting raw material for the production of 1,3-propanediol, has anti-inflammatory action (SZÉL et al., 2015).

Xylitol, a naturally occurring five-carbon polyol (sugar alcohol), is used in various products as a sugar substitute (TAKEUSHI et al., 2018). It is suggested to have beneficial effects on oral health. Chewing gum containing xylitol leads to a reduction in the number of bacteria in saliva, without altering the oral microbial composition, in addition to inhibiting the growth and metabolism of *Streptococcus mutants,* which is the main cause of dental cavities (TAKEUSHI et al., 2018).

Studies showed that 5% concentration xylitol reduced the adhesion of *Streptococcus pneumoniae,* as well as chain formation and increased autolysis of S. *pneumoniae* and *Haemophilus influenzae* in nasopharyngeal epithelial cells (KONTIOKARI et al., 1998).

In cosmetics, xylitol has been used for its moisturizing properties (ANGLENIUS, 2020). However, there are also reports that it inhibits the production of bacterial biofilms, thus reducing the adhesion of these microorganisms (SILVA, 2011).

Studies indicate that a gel containing 5% xylitol associated with 5% glycerol inhibits the growth of *Staphylococcus aureus* and *Staphylococcus pyogenes* both in vitro and in vivo (KORPONYAI, 2017)

A recent study showed that xylitol associated with farnesol inhibits the growth of C. *acnes* and *Staphylococcus aureus,* but a concentration of 5% xylitol to achieve this result (MASAKO, et al. 2005) is needed.

And the evidence of a possible action on sebum control is very weak, just a quote, with no associated scientific results (GUERRERO, 2010).

Regarding keratolytic activity, no evidence of effectiveness of xylitol or sugars of this type was found.

Regarding anti-inflammatory action, studies carried out by SZÉL et al. (2015) disclosed that xylitol at 8.26% and 16.52% has an anti-inflammatory effect, however, both glycerol and xylitol decreased the expression of IL-1β and TNF-α mRNA, but had no effect on the levels IL-1A. These may explain their beneficial effects on cellular inflammation factors, but the exact mechanism through which glycerol and xylitol affect cytokine expression requires further investigation.

Caprylic acid is a saturated fatty acid and one of the constituents of coconut and palm oil. Recently, liposomes loaded with fatty acids were discovered to efficiently inhibit the growth of *C. acnes* by directly fusing into bacterial membranes and releasing acidic cargo. Thus, LIU AND HUANG (2013) conducted a study where they analyzed the inhibitory effects of fatty acids against *C. acnes.* The results showed that only short-chain fatty acids and stearic acid exert a slight inhibition on the growth of *C. acnes.*

There are studies showing that lauric fatty acid inhibits the growth of *C. acnes* when used at a concentration of 80 g/mL and therefore the interest in fatty acids that could have antimicrobial properties is evident, as many of them help to destabilize membranes of microorganisms. Although there are already reports on the effectiveness of caprylic acid for some gram-negative microorganisms, such as *E*. *coli* and *Salmonella,* its effectiveness on other gram-positive microorganisms is considered weak or non-existent (YOON et al., 2018).

A study conducted by HUANG and colleagues (2014) showed that capric acid (C10:0) and lauric acid (C12:0) have antimicrobial and anti-inflammatory activity against *P*. *acne.* This same study indicates that caprylic acid (C8:0), administered at concentrations of 6.9 mM (1 mg/mL) and 8.6 mM (1 mg/mL), had no apparent inhibitory effect on the growth of *C. acnes.* Regarding a possible anti-inflammatory activity of caprylic acid, CHUA and collaborators (2007) compared lipopeptide-based immune-contraceptive vaccines containing different lipid chains and found that the C8:0 chain showed the worst performance in activating NF-κB in a TLR-2 receptor when compared to C18:0, C16:0 and C12:0, which is below the minimum structural requirements for optimal TLR-2 binding and subsequent NF-B signaling.

Regarding keratolytic activity, no evidence of effectiveness of caprylic acid or fatty acids was found.

Xylityl sesquicaprylate is a mixture of mono- and diesters of caprylic acid and hexitol anhydrides derived from xylitol. There is currently no report of specific action of xylityl sesquicaprylate against *C. acnes* or sebum control, keratolytic or anti-inflammatory action.

Studies carried out indicate that the C-8 xylitol monoester has a minimum inhibitory concentration (MIC) between 1.0% and 1.25% against *Staphylococcus aureus, Escherichia coli* and *Candida albicans* and 1.0% and 1.50% against *Pseudomonas aeruginosa* and *Aspergillus niger.* In the same study, a challenge test evaluation of a preservative system was carried out in a lotion containing 1% C-8 xylitol monoester and the result indicated that the molecule has antimicrobial activity against the tested microorganisms, being considered an alternative preservative system for use in cosmetics (AMARAL et al., 2011).

Anhydroxylitol is a byproduct of the synthesis of xylyl caprylate esters. No studies were found reporting antimicrobial activity of anhydroxylitol. There is currently no report of anhydroxylitol specific action against *C. acnes* or sebum control, keratolytic or anti-inflammatory action.

In the search for the state of the art in scientific and patent literature, the following documents were found that deal with the topic:

WO2012151441 - NATURAL ANTIMICROBIAL COMPOSITIONS, which deals with a composition containing a combination of (a) lauric acid, (b) caprylic acid and (c) glycereth-2-cocoate or 1,3-propanediol which acts synergistically against bacteria and fungi, more specifically, *S*. *aureus, P. aeruginosa, E. coli, Candida albicans, Aspergillus niger* and *Phytophthora ramrum* and is indicated for use as a preservation system for cosmetic, pharmaceutical and food products, differing from the use of the object of this patent, which aims application in formulations for sebum control and/or anti-acne treatment.

WO2011047420 - COSMETIC COMPOSITIONS, whose patent subject-matter is an anti-acne, anti-dandruff and anti-dermatitis antimicrobial composition containing (i) a 2-propenal polymer and (ii) a C3-C10 alkanediol, including 1,3-propanediol, which differs from the object of this patent, both in composition and in use for sebum control.

EP2496215 - ANTIMICROBIAL AND ANTI-ACNE FORMULATIONS; wherein glycols are components of the formulation whose main benefit-promoting active ingredient is usnic acid or derivative (usnate).

US20180153177 - BOTANICAL ANTIMICROBIAL COMPOSITIONS wherein 1,3-propanediol is part of a formulation for oral hygiene with microbial control properties, which helps to solubilize the essential oil in order to increase its antimicrobial effectiveness. Document US20180153177 also mentions the use of sugars, one of which is xylitol, as a possible component of the formulation for oral hygiene, but without attributing a specific function related to microbial action to it. Xylitol is commonly used as a sweetener in oral hygiene formulations.

US20090209604 - TOPICAL COMBINATION THERAPY FOR TREATING ACNE wherein the patent object is a composition for acne treatment in which 1,3-propanediol can be one of the chosen glycols to contribute with moisturizing effect.

CA2349698 - SKIN MOISTURIZER COMPOSITIONS CONTAINING A SEBUL CONTROL AGENT wherein fatty acids are described as emulsifying agents of the formulation.

US 8,716,506 B2 and KR101939851 B1 disclose the antimicrobial activities of xylitol derivatives against *Staphylococcus aureus, Escherichia coli, Pseudomonas aeruginosa, Aspergillus niger* and *Candida albicans* and the application as a preservative system for these molecules, associated or not with other antimicrobials. There is no antimicrobial effect on *C. acnes* mentioned, nor a possible use as an aid in controlling acne or sebum production or anti-inflammatory or keratolytic action.

EP1050300 B1 discloses a selective antimicrobial formulation for *S*. *aureus* comprising xylitol and farnesol wherein the concentration of each component is from 0.01 to 30% by weight of xylitol, and from 0.001 to 10% by weight of farnesol. The composition of the present patent application has lower concentrations of polyol due to the synergy between the components, additionally the concentration of polyol in the composition and final formulation of the present patent application are surprisingly lower.

WO2018211333 A1 refers to a preservative system comprising xylitol ester and/or ether, caprylyl glycol, ethylhexylglycerin, and 1,3-propanediol wherein such components can be in the final formulation in a concentration of: 0.1-2, 5% (w/w) xylitol ester and/or ether; 0.25-3.0% (w/w) caprylyl glycol; 0.1-2% (w/w) ethylhexylglycerin; and 1.0-10.0% (w/w) 1,3-propanediol. At no time is a composition as defined in this patent application disclosed, nor its use in the treatment of acne and/or control of sebogenesis.

Thus, from what can be inferred from the researched literature, no documents were found anticipating or suggesting the teachings of the present invention, so that the solution proposed herein has novelty and inventive step compared to the state of the art.

Therefore, there is in the state of the art a need for new antisebogenic compositions, especially antisebogenic compositions that are effective and safe for the control of skin oiliness and for the treatment of acne.

### Summary of the Invention

Thus, the present invention solves the problems of the prior art with an antisebogenic composition comprising polyols, glycols and fatty acids, and the composition can be used to reduce acne-causing bacteria (*Cutibacterium acnes*)*,* control of sebum production and skin oil and minimizing redness and inflammation associated with acne lesions.

In a first object, the present invention presents an antisebogenic composition comprising:
a) 10% to 50% by weight of at least one fatty acid with 3 to 22 carbons;
b) 10% to 50% by weight of at least one polyol;
c) 20% to 70% by weight of at least one glycol.

In a second object, the present invention presents a cosmetic and/or pharmaceutical formulation comprising from 0.1% to 5.0% by weight of said antisebogenic composition and from 0.1% to 15% of a cosmetically and/or pharmaceutically acceptable carrier.

In a third object, the present invention presents the use of said antisebogenic composition in the preparation of a formulation for a dermatological and/or aesthetic method.

These and other objects of the invention will be immediately appreciated by those skilled in the art and will be described in detail below.

### Brief Description of the Figures

The following figures are showed:
Fig. 1 shows a bar graph with the effects of antisebogenic composition, in this embodiment identified as P19/7, on the expression of 5-alpha reductase type 1 in human keratinocytes, cultured after 24 hours of incubation.
Fig. 2 shows a bar graph with the variation in the percentage decrease of porphyrins for P19/7 applied in cosmetic base at 0.5% (w/w) or 1.0% (w/w) and Placebo cosmetic base per period (D7-D0/D28-D0). *p<0.05 in relation to the initial time (D0).
Fig. 3 shows images obtained with the Visia^{®} equipment of two participants illustrating the reduction in porphyrin after 28 days of treatment with P19/7 at 0.5% and 1.0% by weight.
Fig. 4 shows a bar graph with the variation in the percentage of sebum reduction for P19/7 applied in cosmetic base at 0.5% (w/w) or 1.0% (w/w) and Placebo cosmetic base per period (D7-D0/D28-D0). *p<0.05 in relation to the initial time (D0).
Fig. 5 shows a bar graph with the percentage variation in the reduction of inflammatory lesions for P19/7 applied in cosmetic base at 0.5% (w/w) or 1.0% (w/w) and Placebo cosmetic base per period (D7-D0/D28-D0). *p<0.05 in relation to the initial time (D0).
Fig. 6 shows images obtained with the Visia^{®} equipment of two participants illustrating the reduction in inflammatory lesions after 28 days of treatment with P19/7 at 0.5% and 1.0% by weight.
Fig. 7 shows photographs of three participants illustrating the improvement in the appearance of the skin after 28 days of treatment with P19/7 at 0.5% or 1.0% by weight.

### Detailed Description of the Invention

It is a fact that there are a multitude of raw materials on the market aimed at oil control, acne control or to be used as an adjuvant in acne control.

These raw materials could be divided into classical agents such as benzoyl peroxide, salicylic acid, sulfur, resorcinol and adjuvant agents. In general, classic agents are found on positive lists of use when the claim "acne treatment" is sought and, depending on the country and its current legislation, their presence is mandatory in anti-acne products.

In the case of mild acne (grade I and II) the treatment is considered cosmetic. In one embodiment, the composition of the present invention acts in cases of mild acne or in cases of severe acne (grades III to V).

A survey of products currently available on the market indicated that the effectiveness of each product may be associated with one or more acne-related processes.

In one embodiment, the formulations of the present invention act multifunctionally to combat acne as they have proven antimicrobial, sebum-regulating and anti-inflammatory action (minimizes redness associated with acne lesions).

Compared to direct competitors, the formulations of the present invention have greater efficacy results in reducing acne and/or sebum reduction and in shorter times than other products. Among the results, the most innovative and expressive are related to sebum control, an action considered unprecedented/unexpected for the compositions of the present invention.

Briefly, the benefits of the formulations object of the patent are:
- Reduction of acne-causing bacteria in 1 minute (in vitro tests - time kill);
- Quick and noticeable effect from 7 days onwards;
- 53% reduction in acne in 7 days, reaching 73% improvement in 14 days;
- 47% reduction in the average size of acne lesions in 7 days, reaching 60% improvement in 14 days;
- Clinically reduces the presence of porphyrins (byproduct of *C*. *acnes*) by 43% in 7 days;
- Reduces skin oiliness by 15% in 7 days, achieving a 20% reduction in 28 days;
- Reduces redness and inflamed lesions by 15% in 14 days;
- Optionally, formulations of plant origin obtained according to green chemistry precepts; no testing on animals, vegan, aligned with international sustainability standards to obtain products with the lowest environmental impact and chain traceability.

In one embodiment, the composition of the present invention is formed by the synergistic combination of glycols, fatty acids, polyols or sugar alcohols and/or esters and ethers derived from fatty acids and sugar alcohols. In one embodiment, the present invention decreases the necessary amount of xylitol (or its derivatives) to combat acne. Furthermore, the present invention has several advantages (mentioned above) compared to known compositions comprising xylitol and derivatives.

In a first object, the present invention shows an antisebogenic composition comprising:
a) 10% to 50% by weight of at least one fatty acid with 3 to 22 carbons;
b) 10% to 50% by weight of at least one polyol;
c) 20% to 70% by weight of at least one glycol.

In one embodiment, the composition comprises from 20% to 50% by weight of glycol. In one embodiment, the composition comprises from 30% to 50% by weight of glycol. In one embodiment, the composition comprises from 25% to 50% by weight of glycol. In one embodiment, the composition comprises from 25% to 40% by weight of glycol. In one embodiment, the composition comprises 30% to 40% by weight of glycol.

In one embodiment, the glycols are cyclic and/or linear C1-20 chain alkanediols (1 to 20 carbons) and/or C1-20 chain phenyl alcohols. In one embodiment, the glycols are cyclic and/or linear C1-16 chain alkanediols. In one embodiment, the glycols are cyclic and/or linear C1-12 chain alkanediols. In one embodiment, the glycols are cyclic and/or linear C1-10 chain alkanediols. In one embodiment, the glycols are cyclic and/or linear C1-08 chain alkanediols. In one embodiment, the glycols are cyclic and/or linear C1-06 chain alkanediols. In one embodiment, the glycol is 1,3-propanediol, pentylene glycol, hexanediol, caprylyl glycol, benzyl glycol, arachidyl glycol, butylene glycol, cetyl glycol, diethylene glycol, hexacosyl glycol, hexylene glycol, phenylpropanol, phenethyl alcohol, ethoxydiglycol or combinations thereof.

In one embodiment, the fatty acid has a chain of 6 to 20 carbons. In one embodiment, the fatty acid has a chain of 8 to 20 carbons. In one embodiment, the fatty acid has a chain of 3 to 15 carbons. In one embodiment, the fatty acid has a chain of 3 to 10 carbons. In one embodiment, the fatty acid has a chain of 6 to 15 carbons. In one embodiment, the fatty acid has a chain of 6 to 12 carbons. In one embodiment, the fatty acid is selected from the group consisting of butyric acid, caprolic acid, caprylic acid, capric acid, stearic acid, palmitic acid, palmitoleic acid, lauric acid, myristic acid, myristoleic acid, isostearic acid, hydroxystearic acid, oleic acid, linolic acid, linolenic acid, ricinoleic acid, arachidic acid, arachidonic acid, behenic acid and erucic acid. In one embodiment, the fatty acid is caprylic acid. In one embodiment, the composition comprises from 15% to 45% by weight of fatty acid. In one embodiment, the composition comprises from 20% to 40% by weight of fatty acid. In one embodiment, the composition comprises from 25% to 40% by weight of fatty acid. In one embodiment, the composition comprises from 25% to 35% by weight of fatty acid. In one embodiment, the composition comprises 25% to 30% by weight of fatty acid.

In one embodiment, the composition comprises from 20% to 50% by weight of polyol. In one embodiment, the composition comprises from 30% to 50% by weight of polyol. In one embodiment, the composition comprises from 20% to 45% by weight of polyol. In one embodiment, the composition comprises from 30% to 45% by weight of polyol. In one embodiment, the polyols are cyclic and/or linear with 5 or more carbons or are derived from saccharide-type polyols. In one embodiment, the polyols are cyclic and/or linear, have 6 or more carbons and/or are derived from disaccharide-type polyols. In one embodiment, the polyol is xylitol, sorbitol, glycerol, mannitol, maltitol, inositol, derivatives and/or combinations thereof. The esters and ethers derived from the association of fatty acids and polyols can be, more specifically, xylithyl sesquicaprylate and anhydroxylitol.

In a second object, the present invention shows a cosmetic and/or pharmaceutical formulation comprising from 0.1% to 5.0% by weight of said antisebogenic composition and from 0.1% to 30% by weight of a cosmetically and/or pharmaceutically acceptable carrier.

In one embodiment, the formulation comprises from 0.1% to 4.0% by weight of said antisebogenic composition. In one embodiment, the formulation comprises from 0.1% to 3.0% by weight of said antisebogenic composition. In one embodiment, the formulation comprises from 0.1% to 2.0% by weight of said antisebogenic composition. In one embodiment, the formulation comprises from 0.5% to 4.0% by weight of said antisebogenic composition. In one embodiment, the formulation comprises from 0.5% to 3.0% by weight of said antisebogenic composition. In one embodiment, the formulation comprises from 0.5% to 2.0% by weight of said antisebogenic composition. In one embodiment, the formulation comprises from 0.5% to 1.0% by weight of said antisebogenic composition.

In one embodiment, the cosmetically and/or pharmaceutically acceptable carrier is selected from the group consisting of suspending bases, antimicrobial agents, solubilizing agents, anti-acne agents, neutralizing agents, sensory modifiers, fragrances, dyes, pigments and combinations thereof.

In one embodiment, the formulation may be presented as powders, creams, ointments, lotions, sprays, suspensions, gel, cream-gel, microemulsion, nano-emulsion, deep cleansing soap, gentle cleansing soap, moisturizers, sunscreens, mask, oil, wipes, ointments.

In one embodiment, the formulation comprises from 0.1% to 15% by weight of a suspending base, the suspending base being a thickening agent, surfactants or emulsifiers. In one embodiment, the formulation comprises from 0.1% to 10% by weight of a suspending base. In one embodiment, the formulation comprises from 0.1% to 5% by weight of a suspending base. In one embodiment, the formulation comprises from 0.5% to 15% by weight of a suspending base. In one embodiment, the formulation comprises from 0.5% to 10% by weight of a suspending base. In one embodiment, the formulation comprises from 0.5% to 5% by weight of a suspending base.

In one embodiment, the suspending base is a thickening agent selected from the group consisting of sodium polyacrylate, sodium acryloyldimethyl taurate/sodium acrylate copolymer, carbomer, hydroxyethyl cellulose, guar gum, xanthan gum, tara gum, acacia gum, or combinations thereof.

In one embodiment, the suspending base is a surfactant. In one embodiment, the surfactant is selected from the group consisting of sodium lauryl sarcosinate, cocamidopropyl betaine, lauryl glucoside, polysorbate 20, polysorbate 60, polysorbate 80, or combinations thereof.

In one embodiment, the formulation comprises 0.1% to 10.0% by weight of antimicrobial agents for preserving the formulation. In one embodiment, the formulation comprises 0.1% to 5.0% by weight of antimicrobial agents. In one embodiment, the formulation comprises 0.1% to 2.0% by weight of antimicrobial agents. In one embodiment, the antimicrobial agent is benzyl alcohol, potassium sorbate or phenoxyethanol, caprylyl glycol, xylityl sesquicaprylate, sodium anisate, sodium levulinate, ethylhexylglycerin, or combinations thereof.

In one embodiment, the formulation comprises 0.10% to 30% by weight of active solubilizing agents. In one embodiment, the solubilizing agent is ethanol, propylene glycol, ethoxydiglycol, butylene glycol or glycerin.

In one embodiment, the formulation comprises 0.1% to 10% by weight of at least one neutralizing agent. In one embodiment, the formulation comprises 0.1% to 5% by weight of at least one neutralizing agent. In one embodiment, the neutralizing agent is selected from the group consisting of sodium hydroxide, potassium hydroxide, citric acid, arginine, triethanolamine, aminomethylpropanol, 3-amino, 1,2-propanediol, dimethylglucamine, or combinations thereof.

In one embodiment, the formulation comprises 0.1 to 10.0% by weight of classical anti-acne agents. In one embodiment, the anti-acne agent is salicylic acid, benzoyl peroxide, sulfur, sodium sulfacetamide, alpha-hydroxy acids (AHAs), niacinamide, retinoids, plant extracts or combinations thereof.

In one embodiment, the formulation comprises from 0.1% to 20% by weight of emollient. In one embodiment, the formulation comprises from 0.1% to 15% by weight of emollient. In one embodiment, the formulation comprises from 0.1% to 10% by weight of emollient. In one embodiment, the emollient is selected from the group consisting of glycerin, propylene glycol, vegetable oils, fatty acid esters, or combinations thereof.

In a third object, the present invention shows the use of said antisebogenic composition in the preparation of a formulation for a dermatological and/or aesthetic method.

In one embodiment, the antisebogenic composition is used in the preparation of a medicine for treating acne. In one embodiment, the antisebogenic composition is used in the preparation of a formulation for treating skin oiliness. In one embodiment, the antisebogenic composition is used in the preparation of a formulation for treating aging skin; sebaceous hyperplasia; dermatosis; and seborrhea.

In one embodiment, the present invention presents a method of controlling oiliness in the skin, comprising the topical application of an antisebogenic composition as defined above.

In one embodiment, the antisebogenic composition is used in the preparation of a cosmetic and/or dermatological formulation. In one embodiment, the formulation is pharmaceutical.

In one embodiment, the process for producing the composition of the present patent application comprises the steps of: mixing the fatty acid with the polyol under heating; neutralization; filtration; addition of glycol; stirring under heating; and filtration.

In one embodiment, the composition of the present invention (P19/7) can be used in a concentration range between 0.1% to 5.0% by weight, preferably between 0.5 to 1.0% by weight, applied to a cosmetic or pharmaceutical formulation suitable for human or veterinary use.

In one embodiment, the present invention provides a method of controlling acne. In one embodiment, the present invention shows a method of modulating sebogenesis.

As cosmetic or pharmaceutical formulation suitable for human or veterinary use, it is understood any and all type of solvent, dispersion medium, encapsulation, permeation improving agents, surfactants, preservatives and other ingredients, alone or in combination, and which are physiologically compatible.

The compositions and formulations can be used as cosmetics or cosmeceuticals or adjuvants in pharmaceutical products for topical use, or even in products for veterinary use to control oiliness, control or treatment of acne or acne-prone conditions.

This list of applications includes products for oil control, anti-acne or acne treatment products, skin cleansing products, such as deep cleansing soaps, gentle cleansing soaps, gels, lotions, micellar waters, makeup removers, etc., cosmetics for modulation of the microbiota and/or oiliness of the skin and scalp, astringent lotions, facial masks, exfoliating cosmetics, among others.

Thus, the present invention shows a synergistic composition that even at low concentrations in cosmetic and/or pharmaceutical formulations has high efficacy, stability, safety and compatibility with the skin. Furthermore, the components can be of vegetable origin, making it a vegan, green and sustainable option.

### Examples

The examples shown here are intended only to exemplify one of the countless ways of carrying out the invention, however without limiting its scope.

The construction of the product so that the desired efficacy of acne control was achieved went through stages of evaluation of each process involved in the acne condition.

Therefore, for primary evaluation of antimicrobial efficacy, in vitro MIC and Time Kill efficacy tests were performed. An in vitro efficacy test was also carried out to evaluate the expression of 5-alpha reductase, an enzyme involved in the sebogenesis process.

In a second stage, clinical efficacy tests were also carried out, wherein the antimicrobial activity was evaluated through the quantification of porphyrins, a byproduct of the metabolism of C. acnes, the action on sebogenesis, through the oil reduction potential and the anti-inflammatory action, by reducing inflammatory lesions.

### Minimum Inhibitory Concentration - MIC

MIC is defined as the lowest concentration of an antimicrobial that inhibits the visible growth of a microorganism after incubation for a certain period of time. This study aimed to determine the ideal concentration to inhibit the visible growth of *Cutibacterium acnes* and *Staphylococcus aureus.*

The methodology used was based on CLSI - Methods for Dilution Antimicrobial Susceptibility Test for Bacteria that Grow Aerobically M7-A6 - Approved Standard - 10^{th}

P19/7 had an MIC of 0.125 % by weight for *C. acnes* (ATCC6919) and 0.250 % by weight for *S*. *aureus* (ATCC6538).

### Time Kill

The Time-Kill kinetic test, also known as "suspension test or suspension time elimination analysis", determines the time required for a given concentration of antimicrobial agent to kill the microorganism. The main purpose of this test is to evaluate the microbial reduction of the assay after being exposed to the antimicrobial agent.

The methodology used was based on ASTM E2315-16. Standard Guide for Assessment of Antimicrobial Activity Using a Time Kill Procedure.

The microbial reduction potential of *Cutibacterium acnes* (ATCC6919) of a formulation containing P19/7 (diluted in glycerin) at 0.5% by weight and 1.0% by weight was evaluated at contact times of 0, 1, 2, 3, 5, 30, 60 and 90 minutes.

In a Time-Kill test, an antimicrobial agent whose microbial growth reduction is greater than or equal to 99.9% is considered effective, with a reduction of 3-log₁₀ being the minimum level to indicate bactericidal or fungicidal activity against a microorganism. P19/7 at a concentration of 0.5% by weight and 1.0% by weight achieved the expected minimum reduction of 3-log₁₀ in the microbial population from 1 minute of contact of the composition of the present invention with the target microorganism. There was no resumption of microbial growth until the end of the study, at 90 minutes.

### Expression of 5-alpha reductase

The objective of the test was to evaluate the effects of P19/7 on the expression of 5-alpha reductase type 1 in cultured human keratinocytes. The enzyme 5-alpha-reductase (5a-DHT) acts in the conversion of testosterone into dihydrotestosterone (DHT), a steroid responsible for modulating sebaceous secretion.

To carry out the assay, commercially obtained human keratinocytes (Rio de Janeiro Cell Bank - BCRJ) were cultivated in specific culture media and maintained in a humid incubator at 37 °C containing 5% CO₂. When they reached confluence (3rd to 5th passage), cells were seeded in a 6-well plate and incubated with 0.0049% (w/v) P19/7 + Testosterone (1.10⁻⁸ mol/L) or Testosterone (1.10⁻⁸ mol/L) for a period of 24 hours. After 24 hours of incubation, RNA was extracted using the PureLink RNA Mini Kit (Thermo Fisher) and quantified using the NanoDrop Lite spectrophotometer (Thermo Scientific). Tests were performed on a StepOnePlus device (Applied Byosystems). To perform real-time PCR, a commercially available analysis system was used (TaqMan^{®} Gene Expression Assays; SRD5A1: (HS00971643_G1, 5-alpha reductase type 1); B2M: Hs00984230_m1; Applied Biosystems), with primers and probes based on sequence consensus using the TaqMan^{®} RNA-to-CT^{™} 1-step kit (Applied Byosystems). The B2M gene (beta-2-microglobulin) was used as a reference (endogenous). Gene expression of 5-alpha reductase was calculated according to Pfaffl (2001).

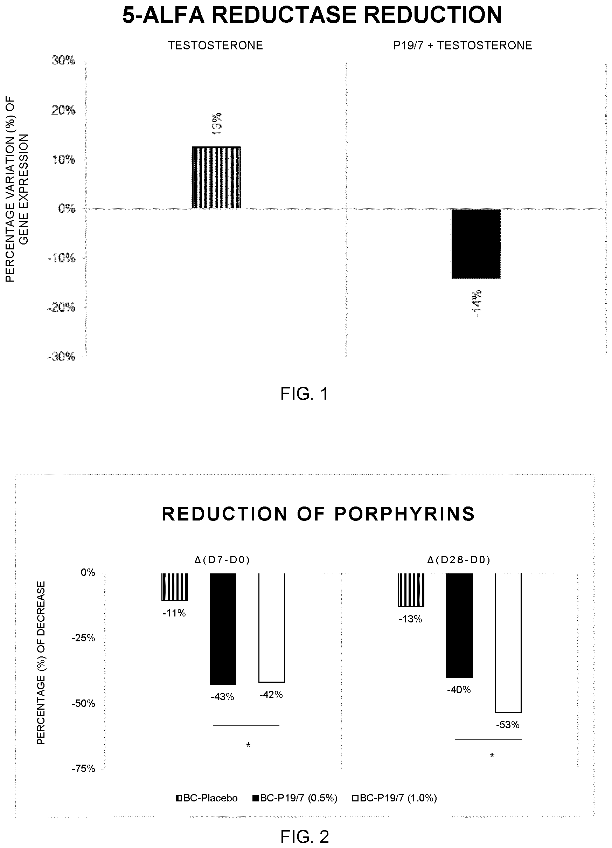

P19/7, under testosterone stress, was able to significantly reduce (P<0.01, with respect to the testosterone control) the relative expression of type 1 5-alpha-reductase by 14%, while the positive testosterone control showed an increase of 13% (P<0.05), compared to the baseline control (Fig. 1).

### Clinical trials

The clinical trials were carried out with 22 volunteers of both sexes, aged between 18 and 30 years, with mixed or oily skin, with mild grade I or II *acne vulgaris,* who used the product twice a day (morning and night), for 28 days. The application of the composition to the skin was enough to cover half of the volunteers' faces. Participants remained in the laboratory for a minimum period of 20 min with controlled temperature and humidity (20 ±2 °C and 50 ±5%), and these conditions were maintained throughout the study period by a properly calibrated thermohygrometer.

To evaluate the composition of the present patent application, the composition P19/7 was applied to a cosmetic base (BC) at a concentration of 0.5% by weight and 1% by weight and compared to a placebo base, without active ingredient (Table 1).

**Table 1. Formulation of samples analyzed:**

| Cosmetic Base Components | % (by weight) Components | | | Function |
|---|---|---|---|---|
| | BC-Placebo | BC-P19/7 0.5% | BC-P19/7 1.0% | |
| Water | q.s. 100,00 | q.s. 100,00 | q.s. 100,00 | Solvent |
| Cetostearyl alcohol (and) Polysorbate 60 | 12 | 12 | 12 | Emulsifier |
| P19/7 | - | 0.5 | 1.0 | Active ingredient |
| Phenoxyethanol | 0.35 | 0.35 | 0.35 | Preservative |
| Potassium sorbate | 0.25 | 0.25 | 0.25 | Preservative |

### Reduction of Porphyrins

Porphyrins are secreted by *C. acnes* and, when exposed to UV light, they absorb photons and pass to an excited singlet state, from which, upon returning to the ground state, they emit fluorescence, allowing their quantification. For this reason, it is an excellent indicator of the presence of *C. acnes.*

The quantification of porphyrins to evaluate the antimicrobial efficacy of P19/7 was carried out by acquiring images on the sides and frontal areas of the volunteers faces using the Visia^{®} - Complexion Analysis equipment (Canfield) using ultraviolet lighting to photograph the most superficial layers of the face and, through the equipment's software, for the quantification itself. The porphyrin count of BC-P19/7 was compared to BC-Placebo before (D0) and after 7 (D7), 14 (D14) and 28 (D28) days of continuous use.

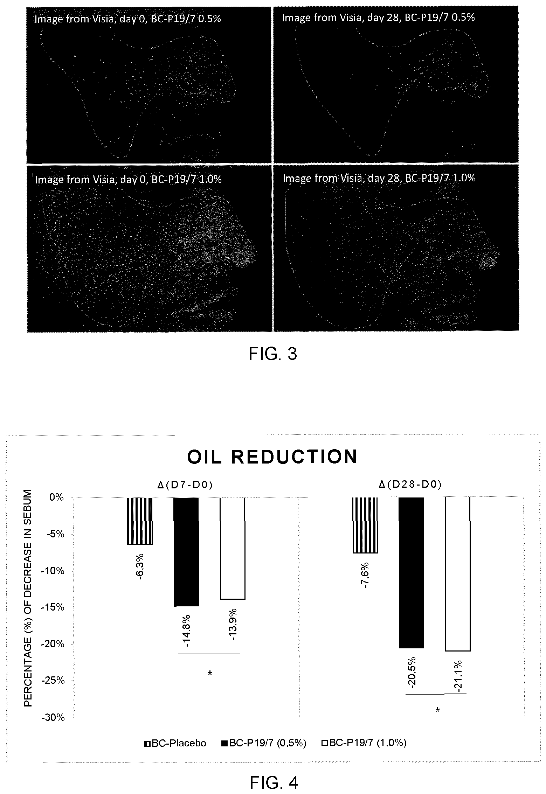

The use of BC-P19/7 (0.5% by weight) and BC-P19/7 (1.0% by weight) led to a significant reduction (p<0.05) in the amount of porphyrins in relation to time initial (D0) at all evaluated times, reaching average reduction levels of 40% for BC-P19/7 (0.5% by weight) and 53.3% for BC-P19/7 (1.0% by weight). weight) in 28 days. No significant reduction in the number of porphyrins was observed for BC-Placebo (Figs. 2 and 3).

### Facial oil reduction

The effectiveness in controlling facial oiliness of BC-P19/7 compared to BC-Placebo was evaluated by taking baseline sebumetry measurements (sebumetric index) in triplicate on the participants' hemifaces before (D0) and after 7 (D7), 14 (D14) and 28 (D28) days of continuous use of the formulations, using Sebumeter^{®} SM 815 equipment (Courage & Khazaka).

The use of BC-P19/7 (0.5% by weight) and BC-P19/7 (1.0% by weight) led to a significant reduction (p<0.05) in skin oiliness over initial time (D0) at all evaluated times, reaching an average level of 20.5% for BC-P19/7 (0.5% in weight) and 21.1% for BC-P19/7 (1.0% in weight) of reduction in 28 days. No significant reduction in skin oil was observed with the use of BC-Placebo (Fig. 4).

### Reduction of Inflammatory Lesions

The quantification of inflammatory lesions/red areas, to evaluate the anti-inflammatory efficacy of P19/7, was done through the acquisition of images on the sides and frontal areas of the volunteers faces using the Visia^{®} - Complexion Analysis (Canfield) equipment using ultraviolet lighting to photograph the most superficial layers of the face and, through the equipment's software, for the quantification itself. The lesion count of BC-P19/7 was compared to BC-Placebo before (D0) and after 7 (D7), 14 (D14) and 28 (D28) days of continuous use.

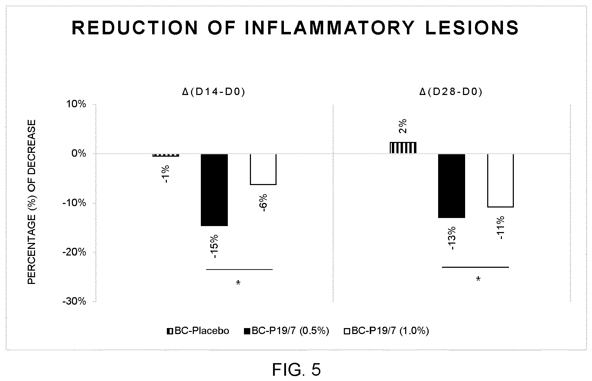

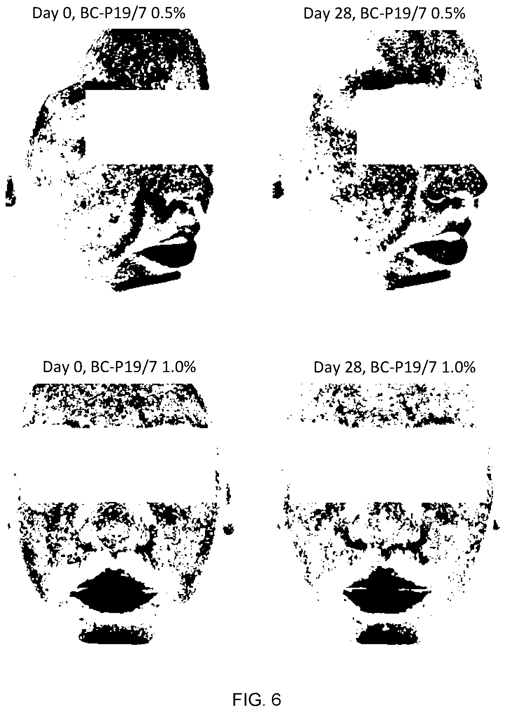

The evaluation of inflammatory lesions indicates a significant reduction (p<0.05) after 14 days of continuous use of BC-P19/7, reaching levels of 12.9% and 10.8% reduction after 28 days respectively, BC-P19/7 (0.5% by weight), BC-P19/7 (1.0% by weight). It is interesting to note that within 28 days, the use of BC-Placebo appears to influence the appearance of more inflammatory lesions. No significant reductions in inflammatory lesions/red areas were observed after 7 (D7), 14 (D14) and 28 (D28) days of BC-Placebo use (Figs. 5 and 6).

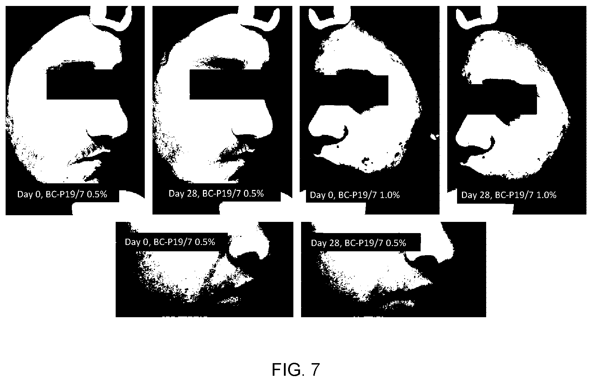

Fig. 7 illustrates the most relevant results of the general appearance of the skin treated with P19/7 at 0.5% and 1.0% by weight for 28 days. Participant TR01 had improvement in the general aspects of the skin (erythema, edema, presence and size of acne) after 28 days of treatment with P19/7 at 0.5% by weight. Similar results were observed for participant TR022, treated with P19/7 at 1.0% by weight. Participant TR07, treated with P19/7 at 0.5% by weight, showed improvement in the acne size parameter for the general appearance of the skin.

According to the dermatologist, the use of P19/7 at 0.5% by weight for 14 and 28 days improved the general appearance of the skin and the presence and size of acne. When P19/7 was at 1.0% by weight, the dermatologist observed improvements in the presence and size of acne after 14 and 28 days. The overall appearance of the skin improved after 28 days of treatment. According to the physician assessment (dermatologist), treatments with P19/7 in both concentrations had excellent and similar performance.

Those skilled in the art will appreciate the knowledge presented herein and will be able to reproduce the invention in the presented embodiments and in other variants and alternatives, covered by the scope of the following claims.

## Claims

1. An antisebogenic composition **characterized in that** it comprises:
a) 10% to 50% by weight of at least one fatty acid with 3 to 22 carbons;
b) 10% to 50% by weight of at least one polyol;
c) 20% to 70% by weight of at least one glycol.

2. The antisebogenic composition, according to claim 1, **characterized in that** it comprises:
a) 25% to 40% by weight of at least one fatty acid with 3 to 10 carbons;
b) 30% to 50% by weight of at least one polyol;
c) 30% to 50% by weight of at least one glycol.

3. The composition according to claim 1 or 2, **characterized in that** the fatty acid is selected from the group consisting of butyric acid, caprolic acid, caprylic acid, capric acid, stearic acid, palmitic acid, palmitoleic acid, lauric acid, myristic acid, myristoleic acid, isostearic acid, hydroxystearic acid, oleic acid, linolic acid, linolenic acid, ricinoleic acid, arachidic acid, arachidonic acid, behenic acid and erucic acid.

4. The composition according to any one of the preceding claims, **characterized in that** the polyol is selected from the group consisting of cyclic and/or linear polyols with 6 or more carbons and disaccharide-type polyol derivatives.

5. The composition, according to claim 4, **characterized in that** the polyol is xylitol, sorbitol, glycerol, mannitol, maltitol, inositol, derivatives and/or combinations thereof.

6. The composition, according to claim 5, **characterized in that** the xylitol derivatives are xylityl sesquicaprylate and anhydroxylitol.

7. The composition, according to any of the previous claims, **characterized in that** the glycol is a cyclic and/or linear C1-20 chain alkanediol.

8. The composition according to claim 7, **characterized in that** the glycol is 1,3-propanediol, pentylene glycol, hexanediol, caprylyl glycol, benzyl glycol, arachidyl glycol, butylene glycol, cetyl glycol, diethylene glycol, hexacosyl glycol, hexylene glycol, phenylpropanol, phenethyl alcohol and/or ethoxydiglycol.

9. A cosmetic and/or pharmaceutical formulation **characterized in that** it comprises from 0.1% to 5.0% by weight of an antisebogenic composition, as defined in any of the previous claims, and from 0.5% to 15% by weight of a cosmetically and/or pharmaceutically acceptable carrier.

10. The formulation according to claim 9, **characterized in that** it comprises from 0.5% to 1.0% by weight of said composition.

11. Use of a composition, as defined in any one of claims 1 to 8, **characterized in that** it is in a dermatological and/or aesthetic treatment.

12. Use of a composition according to claim 11, **characterized in that** it is in the preparation of a medicine for treating acne or sebogenesis.

13. Use of a composition, as defined in any one of claims 1 to 8, **characterized in that** it is for modulating sebogenesis.

14. Use of a composition according to any one of claims 10 to 13, **characterized in that** it is in the preparation of a formulation as defined in claim 9 or 10.
